# EUROPEAN PATENT APPLICATION

(11) **EP 2 672 251 A1**
(43) Date of publication of application: **11.12.2013**
(21) Application number: 13170343.1
(22) Date of filing: 04.06.2013
(51) Int. Cl.: G01N 21/47, G01N 33/553, G01N 21/65

(54) **Sensor for detection of a target of interest**

(30) Priority: 06.06.2012 US 201261656354 P; 15.03.2013 US 201313835379
(71) Applicant: National Taiwan University, Taipei 10617 (TW)
(72) Inventor: Lin, Shiming, Taipei (TW); Lee, Si-Chen, Taipei 106 (TW); Chang, Luan-Yin, Taipei (TW)
(74) Representative: 2K Patentanwälte Blasberg Kewitz & Reichel

(57) **Abstract**

An apparatus for binding a target of interest (901) in a sample (900) comprises a substrate (110), a material (111) disposed on the substrate, and a probe (113) disposed on the material, the probe being configured to bind to the target of interest. A sensor for detecting the target of interest comprises said apparatus, a light source, and a light receiver with a detector, wherein the apparatus is located between the light source and the light receiver, and wherein the apparatus is configured such that light emitted by the light source is scattered by the target bound to the probe.

## Description

### BACKGROUND OF THE DISCLOSURE

A biological sensor is an analytical device for detecting a target molecule by interaction of a biomolecule with the target molecule. Compared to culturing or polymerase chain reaction (PCR), a biological sensor can detect the existence of the target molecule within a relatively short time period. Some biological sensors use chromatographic immunoassay techniques. However, these chromatographic immunoassay-based biological sensors have adoption issues. For example, most chromatographic immunoassay-based biological sensors detect antibodies which are generated by the patients after a later stage of infection/disease.

### BRIEF DESCRIPTION OF THE DRAWINGS

Fig. 1 shows an illustrative embodiment of a sensor for detecting a target of interest (e.g., a biomolecule);
Fig. 2A shows an illustrative embodiment of a container of a sensor for detecting a target of interest (e.g., a biomolecule);
Fig. 2B shows an illustrative embodiment of a container of a sensor for detecting a target of interest (e.g., biomolecule);
Fig. 2C shows an illustrative embodiment of a container of a sensor for detecting a target of interest (e.g., a biomolecule);
Fig. 3 shows an illustrative embodiment of a method for making a sensor;
Fig. 4A is a chart illustrating the signal strength during the sensor detecting a 10% Enterovirus 71 diluted sample collected from an infected patient;
Fig. 4B is a chart illustrating the signal strength during the sensor detecting a control sample collected from a healthy person;
Fig. 5A is a chart illustrating the signal strength during the sensor detecting a 10% Influenza A diluted sample collected from an infected patient;
Fig. 5B is a chart illustrating the signal strength during the sensor detecting a control sample collected from a healthy person;
Fig. 6A is a chart illustrating the signal strength during the sensor detecting a 10% Influenza B diluted sample collected from an infected patient; and
Fig. 6B is a chart illustrating the signal strength during the sensor detecting a control sample collected from a healthy person, all arranged in accordance with embodiments of the disclosure.

### SUMMARY

Some embodiments of the present disclosure may generally relate to an apparatus for binding a target of interest. One example apparatus may comprise a substrate, a material disposed on the substrate and a probe disposed on the material and configured to bind to the target of interest. The probe is configured on the material to scatter light emitted from a light source when the target of interest is bound to the probe.

Some additional embodiments of the present disclosure may generally relate to methods for making an apparatus of a sensor for detecting a target of interest. One example method may include providing a material which includes a pattern configured to scatter light or amplify a light scattering associated with the target of interest, disposing the material on a substrate, and disposing a probe configured to interact with the target of interest on the material.

Other embodiments of the present disclosure may generally relate to methods for using an apparatus of a sensor for detecting a target of interest. One example method may include obtaining a first signal based on light transmitted from a light source of the sensor and passed through the apparatus before a sample that potentially comprises the target of interest is placed in the apparatus, obtaining a second signal based on light transmitted from the light source and passed through the apparatus after the sample is placed in the apparatus, and determining whether the target of interest is present in the sample based on a comparison between the first signal and the second signal. A difference in the two signals indicates that the target is present in the sample.

The foregoing summary is illustrative only and is not intended to be in any way limiting. In addition to the illustrative aspects, embodiments, and features described above, further aspects, embodiments, and features will become apparent by reference to the drawings and the following detailed description.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented here. It will be readily understood that the aspects of the present disclosure, as generally described herein, and illustrated in the figures, can be arranged, substituted, combined, and designed in a wide variety of different configurations, all of which are explicitly contemplated and make part of this disclosure.

In this disclosure, the term "probe" generally refers to a substance (e.g., a biomolecule) that is capable of binding to a target of interest (e.g., a biomolecule). For example, a probe may have a binding affinity for the target of about 100 piconewtons to about 500 piconewtons. Nonlimiting examples of probes include antibodies, antibody fragments that retain the ability to bind to the target of interest, nucleic acids (e.g., DNA, RNA, aptamers), antigens, and enzymes. In a sensor as described herein, a single probe may be used that recognizes a single target of interest, or two or more probes may be used that recognize a single target or multiple targets of interest.

The term "target" generally refers to any molecule that is detectable with a sensor as described herein. A target may include, but is not limited to, a biomolecule. Examples of targets that are detectable in the sensors described herein include, but are not limited to, biomolecules (for example, virus, proteins, nucleic acids, carbohydrates, lipids), and other types of molecules (e.g., small molecules) such as, haptens, and toxins. In some embodiments, the target is a biomolecule that is present in a bodily fluid and/or tissue.

In some embodiments, a sensor for detecting a target of interest (e.g., a biomolecule) includes a light source, a container, and a light receiver having a light detector that generates an electrical signal that is proportionate to the amount of light received by the light receiver. At least one inner surface of the container includes probes immobilized on a material that is disposed on the container surface. The light source is configured to generate light that passes through the container and eventually to the light detector. The light may be of a specific wavelength. Depending on the target to be detected, the specific wavelength may be changed accordingly. The specific wavelength may be determined by any technical feasible approaches. In some embodiments, the specific wavelength is determined by scanning the target with the visible light spectrum or UV light spectrum. The maximum absorption wavelength of the target in the visible light spectrum may be the specific wavelength. For example, any of enterovirus 71, influenza A virus, and influenza B virus has a maximum absorption at about 560 nm wavelength in the visible light spectrum and has a relatively greater absorption at about 280 nm wavelength in the ultra-violet spectrum. Adenovirus has a maximum absorption at about 340 nm wavelength in the visible light spectrum and has a relatively greater absorption at about 280 nm wavelength in the ultra-violet spectru. If the target is present and bound to the probes, light is scattered as it passes through the container, amplifying the signal and resulting in a higher level of light reaching the light receiver, and a larger magnitude electrical signal generated by the light detector.

In some embodiments, the light source may generate visible light or ultra-violet light. A light filter may be placed between the light source and the container so that the light entering the container has a specific wavelength. Alternatively, the light filter may be placed between the container and the light detector so that the light entering the light detector has a specific wavelength. Alternatively, some optical elements (e.g., slit, grating, mirror and a linear charge-coupled device) may be placed between the container and the light detector to make the visible light passed through the container turn to a monochromatic light with a specific wavelength before entering the light detector. The light source may also be a monochromatic light source.

In some embodiments, the container includes a substrate, a material disposed on the substrate and one or more probe(s) disposed on the material. The probe is immobilized on the material. The probe may be a biological substance, for example, an antibody, an antibody fragment, a nucleic acid, an aptamer, an antigen or an enzyme, or any substance that is capable of binding to a target of interest in a manner such that light scattering occurs when the target is bound to the probe to a greater degree than when no target is bound. The material is compatible with the probe and the probe can be immobilized on the material. The material may be, for example, a metal such as gold, silver, copper and nickel. The substrate may be composed of any composition that is technically feasible for the material to be disposed thereon, and that does not interfere with detection of a target of interest as described herein. Some examples of suitable substrates may include, without limitation, glass, metal, silicon or polymers.

The material includes a pattern configured to enhance the light scattering when the target is bound to the probe. In some embodiments, the pattern itself is configured to scatter light when the light travels through the pattern. In some embodiments, the pattern may be a film coated on the substrate. In some other embodiments, the coated film may be annealed. The annealing makes the surface of the coated film becomes uneven. The uneven surface may enhance the scattering of the light passing through the container.

In some embodiments, the pattern may be a metal rod array disposed on the substrate. The size of a metal rod in the metal rod array is associated with the specific wavelength set forth above. The length of any metal rod is neither a multiple nor a factor of the specific wavelength. The width of any metal rod is neither a multiple nor a factor of the specific wavelength. The distance between two adjacent rods is neither a multiple nor a factor of the specific wavelength.

The probe is disposed or immobilized on the material through one or more chemical bonds (e.g., covalent bond) with the material. The probe may form a "lock and key" relationship with the target to be detected by the sensor. For example, the probe may be DNA, RNA, a protein, an antibody, an antibody fragment, an aptamer, an antigen, or an enzyme. In some embodiments, the probe is an antibody and the target is an antigen to which the antibody binds.

A sample potentially including the target is introduced into the sensor and then flows over the probe. If the target exists in the sample, the amount of photons passing through the container before the sample is introduced into the sensor may be different than the amount of photons passing through the container after the sample is introduced into the sensor because the target coupled with the probe scatters photons. If the target does not exist in the sample, the amount of photons passing through the container remains substantially the same because there is no bound target to scatter photons. In some embodiments, the amount of photons absorbed by the sample is higher in the presence of bound target than in the absence of the target, resulting in a difference in the light signal detected when the target is present and the light signal detected when the target is absent.

In some embodiments, a method for making a sensor is disclosed. The method includes providing a material which includes a pattern configured to scatter light or enhance a light scattering associated with the target of interest, disposing the material on a substrate and disposing a probe configured to interact with the target of interest on the material.

In some embodiments, the material is a film. The substrate may be cleaned before the material is disposed on the substrate. In some embodiments, before the material is disposed on the substrate, an adhesion layer is disposed on the substrate first. Then the material is disposed on the adhesion layer. The adhesion layer may be chromium.

In some other embodiments, the material is an annealed film which has an uneven surface pattern. The material may be annealed at a temperature from about 300 degrees Celsius to about 500 degrees Celsius after the material is disposed on the adhesion layer.

In some other embodiments, the material includes a rod array pattern. A photoresist is coated on the substrate and a photolithography process is performed to form the rod array. The size of each rod in the rod array is associated with the specific wavelength set forth above. The distance between two adjacent rods is also associated with the specific wavelength.

In some embodiments, a method for detecting a target of interest with a sensor described herein is disclosed. The sensor includes a light source, a container and a light detector. The container includes a substrate, a material disposed on the substrate and a probe configured to interact with the target of interest. The probe is disposed and immobilized on the material. The method includes transmitting light from the light source through the container and obtaining a first signal based on the light received by a light detector of the sensor.

The method also includes placing a sample that potentially includes the target of interest in the sensor and obtaining a second signal based on the light received by the light detector after the sample is placed in the container. The method further includes comparing the first signal and the second signal and determining whether the target exists in the sample based on the comparison.

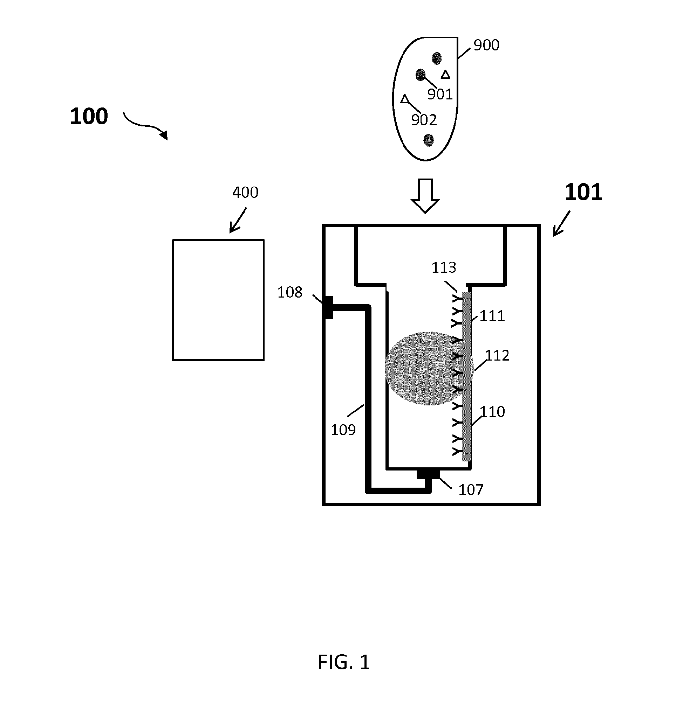

FIG. 1 is an illustrative embodiment of a sensor 100 for detecting a target of interest. The sensor 100 includes a container 101. The container forms an apparatus for binding a target of interest, and includes a material 111 disposed on a substrate 110 (e.g., one or more walls of the container) and probes 113 that are capable of binding to the target disposed on the material. A light 112 is transmitted from a light source of the sensor 100 to a light receiver of the sensor 100 through the container 101. The probes 113 disposed on the material 111 are configured in the path of the light emitted by the light source. The probes 113 are configured such that light passing over the apparatus is scattered when the target of interest is bound to the probes 113.

A solution that does not contain the target, such as a buffer solution, is introduced into the container 101. The light 112 is configured to pass through the container 101 in a first time slot and is received by the light receiver. The light receiver further includes a photodiode detector configured to generate a first electronic signal based on the amount of the light 112 that is received by the light receiver in the first time slot. The light receiver further includes a processor for processing the first electronic signal.

A sample 900 potentially including the target of interest 901 is then introduced into the container 101. A predetermined amount of time is allowed to lapse so that if the target of interest 901 exists in the sample 900, the target of interest 901 may bind to the probes 113 on the material 111. After the predetermined period of time has lapsed, introduced sample 900 in the container 101, including impurities 902, is removed from the container 101 by a draining pump 400 through a first hole 107, a passage 109 and a second hole 108, without breaking the bond between the target of interest 901 and the probes 113. Subsequently, the container 101 is rinsed with a buffer solution for a predetermined number of times. For example, fresh buffer solution may be repeatedly injected into and pumped out from the container 101 several times.

Light scattering that occurs when target molecules are bound to the probes 113 on the apparatus will be detected, indicating presence of the target 901 in the sample 900. The light 112 is configured to pass through the container 101 in a second time slot and is received by the light receiver. The photodiode detector of the light receiver is configured to generate a second electronic signal based on the amount of the light that is received by the light receiver in the second time slot. The processor of the light receiver is configured to further process the second electronic signal. If the second signal is significantly stronger than the first signal, the processor determines that the target of interest is present in the sample.

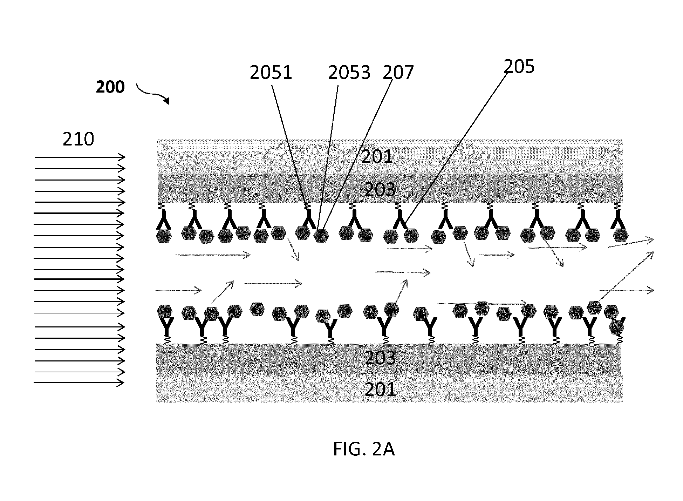

Fig. 2A shows an illustrative embodiment of an apparatus for binding a target of interest. The apparatus 200 includes a substrate 201, a film 203 disposed on the substrate and a probe 205 disposed on the film 203. The thickness of the film 203 may be, for example, a substantially even thickness of about 5nm to about 200nm. A first surface 2051 of the probe 205 is disposed on the material 203. A second surface 2053 of the probe 205 having a binding affinity for the target 207 is configured such that it is available to couple with the target 207 when the target 207 is present. The probes 205 are configured on the film 203 such that light 210 (shown as arrow in Fig. 2A) is scattered when the target 207 binds to the second surface 2053 of probes 205.

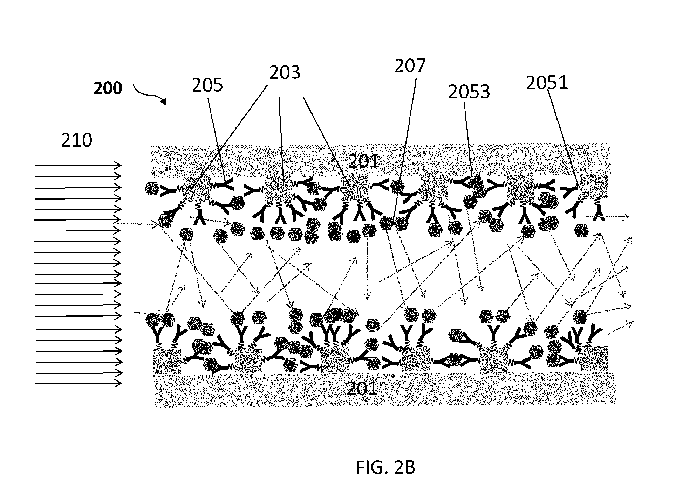

Fig. 2B shows an illustrative embodiment of an apparatus for binding a target of interest. The apparatus 200 includes a substrate 201, a rod array 203 disposed on the substrate and a probe 205 disposed on the rod array 203. The width and the length of any rod in the rod array are associated with the wavelength of light transmitted from a light source of the sensor. The wavelength is specific to a target of interest 207. The distance between two adjacent rods is also associated with such wavelength. In some embodiments, the width, the length and the distance are all neither a multiple of the wavelength, nor a factor of the wavelength. In some embodiments, the rod of the rod array may have a length from 200 to 900 nm, a width from 200 to 900 nm and a height from 15 to 1500 nm. The distance between each rod in the rod array may be from 200 to 900nm. In some embodiments, the rod of the rod array may have a length of approximately 500 nm, a width of approximately 500 nm and a height of about 100 nm, and the distance between each rod may be approximately 500 nm. A first surface 2051 of the probe 205 is disposed on the rod array 203. A second surface 2053 of the probe 205 having a binding affinity for the target 207 is configured such that it is available to couple with the target 207 when the target 207 is present. The probes 205 are configured on the rod array 203 such that light 210 (shown as arrow in Fig. 2B) is scattered when the target 207 binds to the second surface 2053 of probes 205.

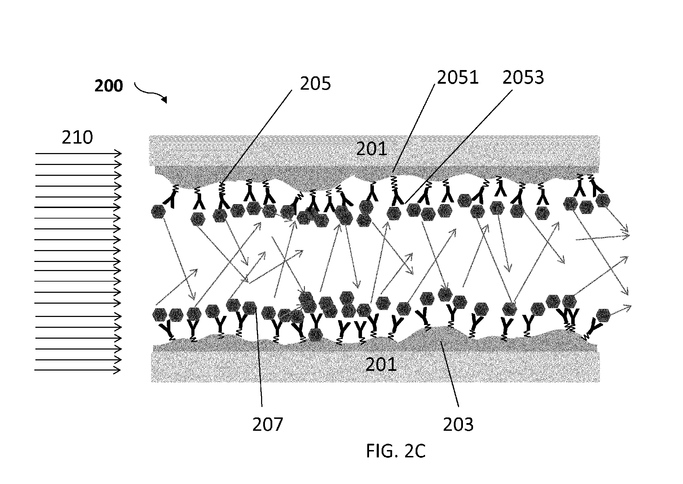

Fig. 2C shows an illustrative embodiment of an apparatus for binding a target of interest. The apparatus 200 includes a substrate 201, a film 203 disposed on the substrate and a probe 205 disposed on the film 203. The film 203 has an uneven thickness. In some embodiments, the film may be first coated on the substrate 201 and then annealed to form the uneven thickness. In some embodiments, the thickness of the film 203 may vary from approximately 0.5nm to approximately 30 nm. A first surface 2051 of the probe 205 is disposed on the film 203. A second surface 2053 of the probe 205 having a binding affinity for the target 207 is configured such that it is available to couple with the target 207 when the target 207 is present. The probes 205 are configured on the material 203 such that light 210 (shown as arrow in Fig. 2C) is scattered when the target 207 binds to the second surface 2053 of probes 205.

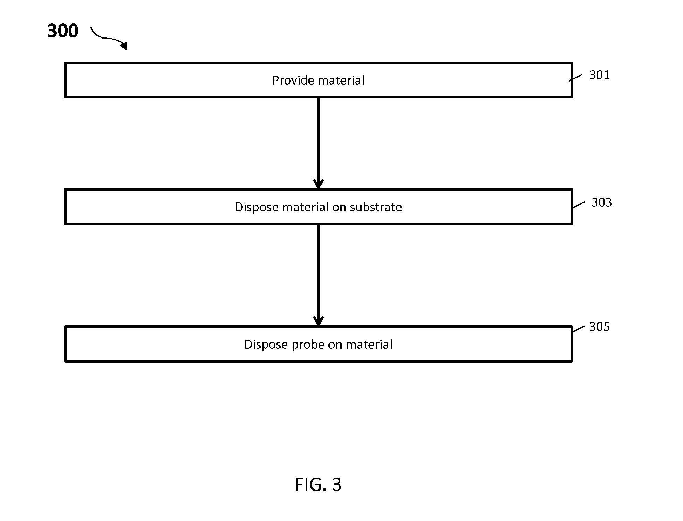

Fig. 3 shows a flow chart of an illustrative embodiment of a method 300 for making an apparatus for binding a target of interest. The method 300 includes steps 301, 303 and 305. In step 301, a material is provided. In step 303, the material is disposed on a substrate. The material may be disposed on the substrate in a pattern configured to permit and/or enhance scattering light emitted from a light source and traveling across the apparatus when a target of interest is bound to a probe that is disposed on the material. The pattern may be, for example, a film, a film with an uneven thickness, or a rod array. In step 305, a probe that is capable of binding to a target of interest is disposed on the material. The probe is configured to interact with the target that the sensor configured to detect. In some embodiments, before disposing the probe on the material, the material may be cleaned and pre-treated. For example, the material may be cleaned with an acidic solution, a basic solution, and/or purified water. In some embodiments, the material may be pretreated with one or more compounds. In one embodiment, the material may be pretreated with one or more compound(s) that include(s) at least one functional group compatible with the material. In another embodiment, the material may be pretreated with one or more compound(s) that include(s) at least one functional group compatible with the probe. The functional group is configured to form a first stable bound with the free electrons around the surface of the material and form a second stable bound with the probe. Some example functional groups include, but not limited to, thiol group and hydroxyl group.

### [Example 1]

### [Probe Immobilization]

A glass container configured to contain a sample was placed in a plastic holder and the glass container and the plastic holder were then placed in *p*Tricorder® sensor (Vsense Medtech. Co., Ltd., Taipei, Taiwan). Gold was disposed on an inner surface of the container in the form of a film having an uneven thickness from about 5nm to about 20nm. Before introducing probes into the container, the gold film was cleaned with a 0.1M hydrochloric acid solution, purified water, 0.1M sodium hydroxide, and purified water, in sequence.

After cleaning, an aqueous solution containing 110µL of cystamine (20mM in phosphate buffered saline (PBS) solution at pH 7.2) was added into the container and incubated for 20 minutes at room temperature to permit cystamine to bind to the gold on the container wall. The remaining cystamine solution was then removed from the container. An aqueous solution containing 110µL of glutaraldehyde (2.5% in PBS solution at pH 7.2) was then added into the container and incubated for 20 minutes at room temperature to permit glutaraldehyde to bind to the cystamine.

After removing the remaining glutaraldehyde solution from the container, an aqueous solution of 110µL of commercial available anti-Enterovirus 71 monoclonal antibodies was added into the container and incubated for 20 minutes at room temperature to permit anti-Enterovirus 71 monoclonal antibodies to bind to the glutaraldehyde crosslinker. Unbound anti-Enterovirus 71 monoclonal antibody was then removed from the container by a draining pump of the sensor through a hole at the bottom of the glass container. An aqueous solution of 0.5M glycine was then added to the container to react with residual unbound glutaraldehyde. Finally, glycine was removed from and PBS was added to the container.

### [Sample Detection]

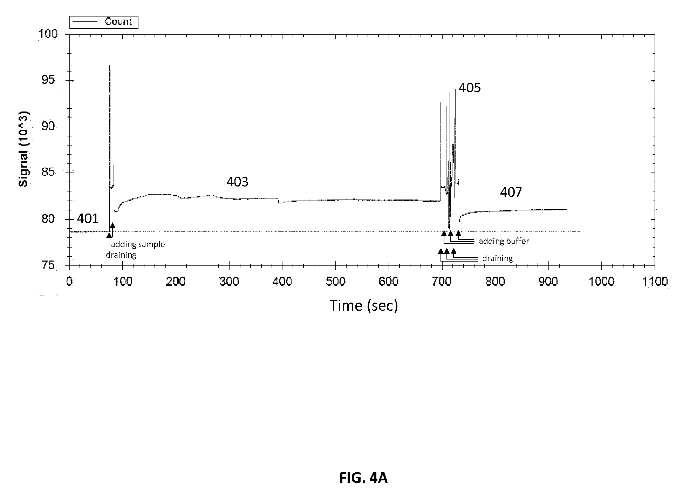

The sensor further includes a visible light source and a light detector for detection of Enterovirus 71. Visible light was transmitted from the visible light source and passed through an optical filter. The optical filter was configured to filter the visible light and only the light having a 560 nm wavelength can pass the optical filter. The light (i.e., having the wavelength of 560 nm) then passed through the glass container set forth above. After passing through the glass container, the light was eventually received by the light detector. FIG. 4A is a chart illustrating the signal strength during detection of Enterovirus 71 in a 10% diluted sample collected from an infected patient. The sample was collected by a throat swab from the infected patient.

The sensor was turned on so that light transmitted from a light source of the sensor passed through the container and over the probes on the inner surface of the container. At this stage, the container contained PBS as set forth above. The signal detected with PBS (as shown at 401 in FIG. 4A) was used as a reference.

After about 1 minute, PBS was removed from the container and the 10% Enterovirus 71 diluted sample was added to the container. Data was collected for 10 minutes. Light detected from the container is shown at 403 in FIG. 4A. After 10 minutes, the Enterovirus 71 diluted sample was removed from the container.

PBS was added to rinse the container to remove nonspecifically bound Enterovirus 71. The rinsing was repeated several times. The rinsing caused various sharp peaks as shown at 405 in FIG. 4A. After rinsing, PBS was added to the container and data was collected for 3 minutes to collect data for light detected from the container (as shown at 407 in FIG. 4A). The difference between the light signal detected at 407 and the signal detected at 401 indicated presence of Enterovirus 71 in the sample.

### [Comparative Example 1]

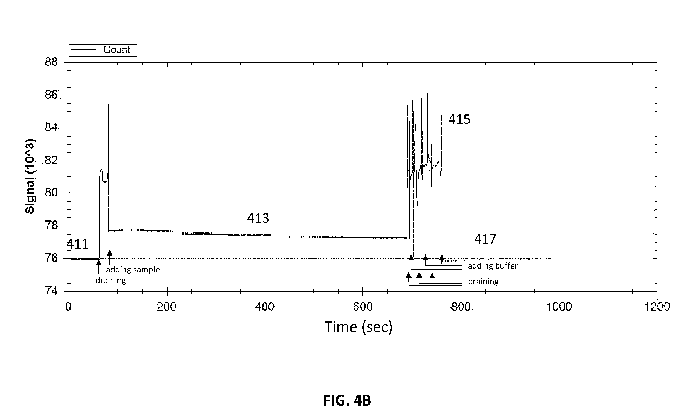

FIG. 4B is a chart illustrating the signal strength during detection of a control sample collected from a healthy person. The detection approach was the same as the approach of the detection of the 10% Enterovirus 71 diluted sample set forth above.

The sensor was turned on so that a light transmitted from a light source of the sensor passed through the container and over the probes on the inner surface of the container. At this stage, the container contained PBS. The signal detected with PBS (as shown at 411 in FIG. 4B) was used as a reference.

After about 1 minute, PBS was removed from the container and the control sample was added to the container. Data was collected for 10 minutes. Light detected from the container is shown at 413 in FIG. 4B. After 10 minutes, the control sample was removed from the container.

PBS was added to rinse the container to remove nonspecifically bound material. The rinsing was repeated several times. The rinsing caused various sharp peaks as shown at 415 in FIG. 4B. After rinsing, PBS was added to the container and data was collected for 3 minutes to collect data for light detected from the container (as shown at 417 in FIG. 4B). The similar signal strength of 411 and 417 showed no existence of Enterovirus 71 in the control sample.

### [Example 2]

### [Probe Immobilization]

A glass container configured to contain a sample was placed in a plastic holder and the glass container and the plastic holder were then placed in *p*Tricorder® sensor (Vsense Medtech, Taipei, Taiwan). Gold was disposed on an inner surface of the container in the form of a film having an uneven thickness from about 5 nm to about 20 nm. Before introducing probes into the container, the gold film was cleaned with a 0.1M hydrochloric acid solution, purified water, 0.1M sodium hydroxide, and purified water, in sequence.

After cleaning, an aqueous solution containing 110µL of cystamine (20mM in phosphate buffered saline (PBS) solution at pH 7.2) was added into the container and incubated for 20 minutes at room temperature to permit cystamine to bind to the gold on the container wall. The remaining cystamine solution was then removed from the container. An aqueous solution containing 110µL of glutaraldehyde (2.5% in PBS solution at pH 7.2) was then added into the container and incubated for 20 minutes at room temperature to permit glutaraldehyde to bind to the cystamine.

After removing the remaining glutaraldehyde solution from the container, an aqueous solution of 110µL of commercially available anti-Influenza A antibody (20µg/ml in PBS solution at pH 7.2) was added into the container and incubated for 20 minutes at room temperature to permit the anti-Influenza A antibody to bind to the glutaraldehyde crosslinker. Unbound anti-Influenza A antibody was then removed from the container by a draining pump of the sensor through a hole at the bottom of the glass container. An aqueous solution of 0.5M glycine was then added to the container to react with residual unbound glutaraldehyde. Finally, glycine was removed from and PBS was added to the container.

### [Sample Detection]

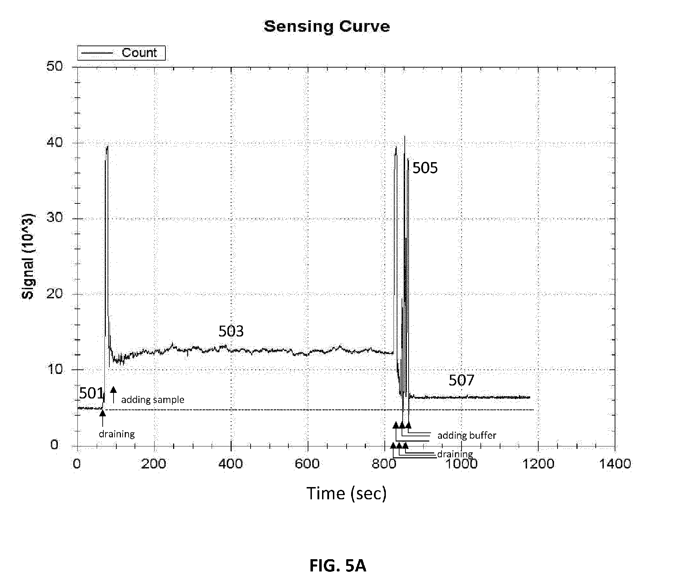

The sensor further includes a visible light source and a light detector for detection of Influenza A. Visible light was transmitted from the visible light source and passed through an optical filter. The optical filter was configured to filter the visible light and only the light having a 560 nm wavelength can pass the optical filter. The light (i.e., having the wavelength of 560 nm) then passed through the glass container set forth above. After passing through the glass container, the light was eventually received by the light detector. FIG. 5A is a chart illustrating the signal strength during detection of Influenza A in a 10% diluted sample collected from an infected patient. The sample was collected by a throat swab from the infected patient's throat.

The sensor was turned on so that light transmitted from a light source of the sensor passed through the container and over the probes on the inner surface of the container. At this stage, the container contained PBS as set forth above. The signal detected with PBS (as shown at 501 in FIG. 5A) was used as a reference.

After about 1 minute, PBS was removed from the container and the 10% Influenza A diluted sample was added to the container. Data was collected for 10 minutes. Light detected from the container is shown at 503 in FIG. 5A. After 10 minutes, the Influenza A diluted sample was removed from the container.

PBS was added to rinse the container to remove nonspecifically bound material. The rinsing was repeated several times. The rinsing caused various sharp peaks as shown at 505 in FIG. 5A. After rinsing, PBS was added to the container and data was collected for 3 minutes to collect data for light detected from the container (as shown at 507 in FIG. 5A). The difference between the light signal detected at 507 and the signal detected at 501 indicated presence of Influenza A in the sample.

### [Comparative Example 2]

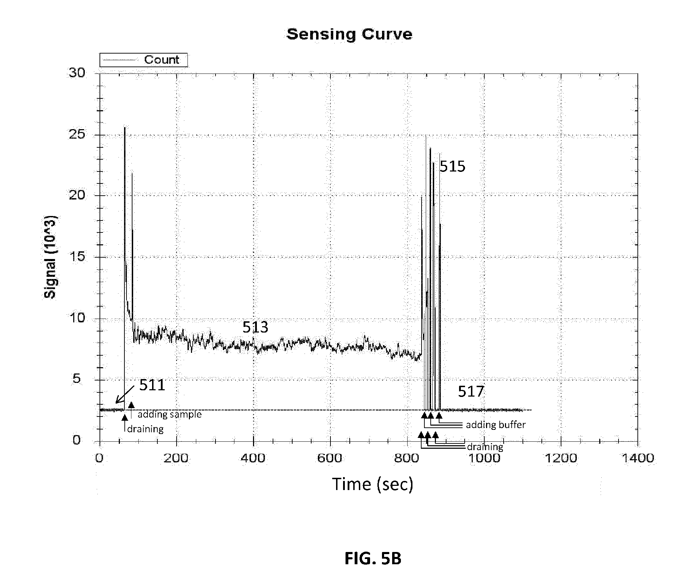

FIG. 5B is a chart illustrating the signal strength during detection of a control sample collected from a healthy person. The detection approach was the same as the approach of the detection of the Influenza A diluted sample set forth above.

The sensor was turned on so that a light transmitted from a light source of the sensor passed through the container and over the probes on the inner surface of the container. At this stage, the container contained PBS. The signal detected with PBS (as shown at 511 in FIG. 5B) was used as a reference.

After about 1 minute, PBS was removed from the container and the control sample was added to the container. Data was collected for 10 minutes. Light detected from the container is shown at 513 in FIG. 5B. After 10 minutes, the control sample was removed from the container.

PBS was added to rinse the container to remove nonspecifically bound material. The rinsing was repeated several times. The rinsing caused various sharp peaks as shown at 515 in FIG. 5B. After rinsing, PBS was added to the container and data was collected for 3 minutes to collect data for light detected from the container (as shown at 517 in FIG. 5B). The similar signal strength of 511 and 517 showed no existence of Influenza A in the control sample.

### [Example 3]

### [Probe Immobilization]

A glass container configured to contain a sample was placed in a plastic holder and the glass container and the plastic holder were then placed in *p*Tricorder® sensor (Vsense Medtech, Taipei, Taiwan). Gold was disposed on an inner surface of the container in the form of a film having an uneven thickness from about 5 nm to about 20 nm. Before introducing probes into the container, the gold film was cleaned with a 0.1M hydrochloric acid solution, purified water, 0.1M sodium hydroxide, and purified water, in sequence.

After cleaning, an aqueous solution containing 110µL of cystamine (20mM in phosphate buffered saline (PBS) solution at pH 7.2) was added into the container and incubated for 20 minutes at room temperature to permit cystamine to bind to the gold on the container wall. The remaining cystamine solution was then removed from the container. An aqueous solution containing 110µL of glutaraldehyde (2.5% in PBS solution at pH 7.2) was then added into the container and incubated for 20 minutes at room temperature to permit glutaraldehyde to bind to the cystamine.

After removing the remaining glutaraldehyde solution from the container, an aqueous solution of 110µL of commercially available anti-Influenza B antibody (20µg/ml in PBS solution at pH 7.2) was added into the container and incubated for 20 minutes at room temperature to permit the anti-Influenza B antibody to bind to the glutaraldehyde crosslinker. Unbound anti-Influenza B antibody was then removed from the container by a draining pump of the sensor through a hole at the bottom of the glass container. An aqueous solution of 0.5M glycine was then added to the container to react with residual unbound glutaraldehyde. Finally, glycine was removed from and PBS was added to the container.

### [Sample Detection]

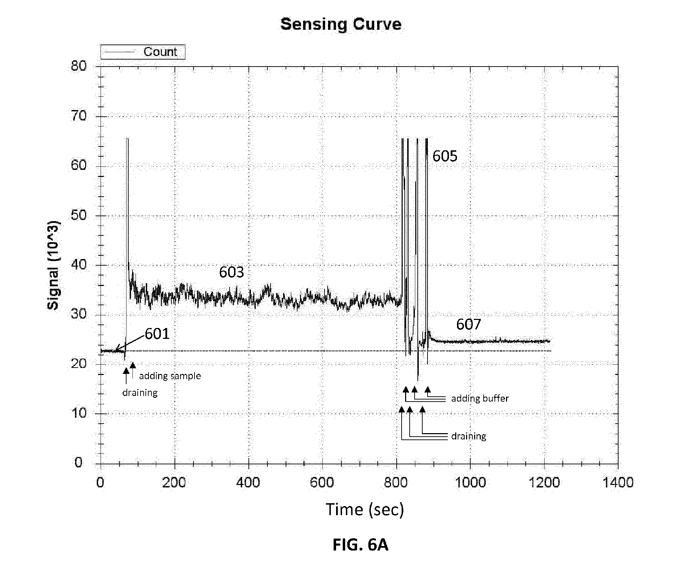

The sensor further includes a visible light source and a light detector for detection of Influenza B. Visible light was transmitted from the visible light source and passed through an optical filter. The optical filter was configured to filter the visible light and only the light having a 560 nm wavelength can pass the optical filter. The light (i.e., having the wavelength of 560 nm) then passed through the glass container set forth above. After passing through the glass container, the light was eventually received by the light detector. FIG. 6A is a chart illustrating the signal strength during detection of Influenza B in a 10% diluted sample collected from an infected patient. The sample was collected by a throat swab from the infected patient's throat.

The sensor was turned on so that light transmitted from a light source of the sensor passed through the container and over the probes on the inner surface of the container. At this stage, the container contained PBS as set forth above. The signal detected with PBS (as shown at 601 in FIG. 6A) was used as a reference.

After about 1 minute, PBS was removed from the container and the 10% Influenza B diluted sample was added to the container. Data was collected for 10 minutes. Light detected from the container is shown at 603 in FIG. 6A. After 10 minutes, the Influenza B diluted sample was removed from the container.

PBS was added to rinse the container to remove nonspecifically bound material. The rinsing was repeated several times. The rinsing caused various sharp peaks as shown at 605 in FIG. 6A. After rinsing, PBS was added to the container and data was collected for 3 minutes to collect data for light detected from the container (as shown at 607 in FIG. 6A). The difference between the light signal detected at 607 and the signal detected at 601 indicated presence of Influenza B in the sample.

### [Comparative Example 3]

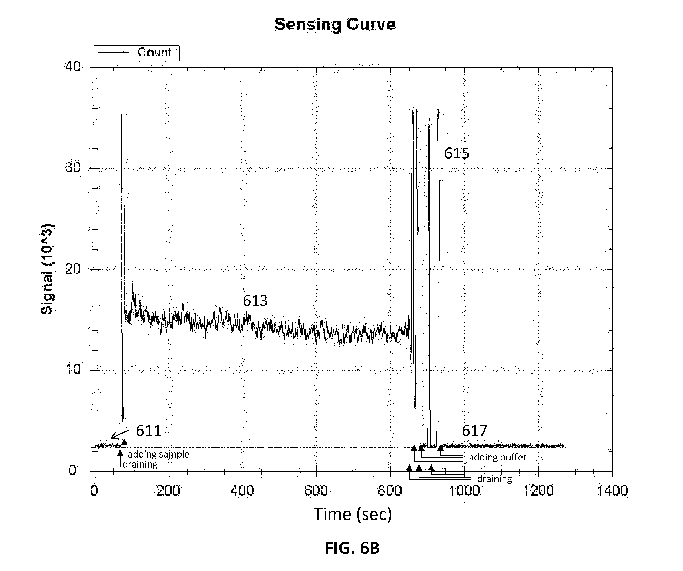

FIG. 6B is a chart illustrating the signal strength during detection of a control sample collected from a healthy person. The detection approach was the same as the approach of the detection of the Influenza B diluted sample set forth above.

The sensor was turned on so that a light transmitted from a light source of the sensor passed through the container and over the probes on the inner surface of the container. At this stage, the container contained PBS. The signal detected with PBS (as shown at 611 in FIG. 6B) was used as a reference.

After about 1 minute, PBS was removed from the container and the control sample was added to the container. Data was collected for 10 minutes. Light detected from the container is shown at 613 in FIG. 6B. After 10 minutes, the control sample was removed from the container.

PBS was added to rinse the container to remove nonspecifically bound material. The rinsing was repeated several times. The rinsing caused various sharp peaks as shown at 615 in FIG. 6B. After rinsing, PBS was added to the container and data was collected for 3 minutes to collect data for light detected from the container (as shown at 617 in FIG. 6B). The similar signal strength of 611 and 617 showed no existence of Influenza B in the control sample.

Although the foregoing invention has been described in some detail by way of illustration and examples for purposes of clarity of understanding, it will be apparent to those skilled in the art that certain changes and modifications may be practiced without departing from the spirit and scope of the invention. Therefore, the description should not be construed as limiting the scope of the invention.

All publications, patents, and patent applications cited herein are hereby incorporated by reference in their entireties for all purposes and to the same extent as if each individual publication, patent, or patent application were specifically and individually indicated to be so incorporated by reference.

## Claims

1. An apparatus for binding a target of interest, comprising:
a substrate;
a material disposed on the substrate; and
a probe disposed on the material and configured to bind to the target of interest, wherein the probe is configured on the material to scatter light emitted from a light source when the target of interest is bound to the probe.

2. The apparatus of claim 1, wherein the probe comprises DNA, RNA, a protein, an antibody, an antibody fragment, an aptamer, an antigen, or an epitope, and/or
wherein the target of interest is a biomolecule.

3. The apparatus of claim 1 or 2, wherein the target comprises a virus, a protein, a nucleic acid, a carbohydrate, a lipid, a hapten, or a toxin.

4. The apparatus of one of the preceding claims, wherein the probe binds to the target of interest with an affinity of about 100 piconewtons to about 500 piconewtons.

5. The apparatus of one of the preceding claims, wherein the material comprises a metal, and/or
wherein the material comprises a pattern configured to scatter light emitted from the light source.

6. The apparatus of claim 5, wherein the pattern is further configured to facilitate the probe to scatter light emitted from the light source when the target of interest is bound to the probe, and/or
wherein the pattern comprises a film on the substrate.

7. The apparatus of claim 6, wherein the film comprises a substantially even thickness or an uneven thickness, in particular a substantially even thickness of about 5nm to 200 nm or an uneven thickness varying from about 0.5 nm to about 30 nm.

8. The apparatus of claim 5, wherein the light emitted from the light source has a wavelength associated with the target of interest.

9. The apparatus of claim 8, wherein the pattern comprises a rod array disposed on the substrate, in particular a rod of the rod array having a length from 200 to 900 nm, a width from 200 to 900 nm and a height from 15 to 1500 nm.

10. The apparatus of claim 9, wherein the size of a rod in the rod array is associated with the wavelength, and/or wherein a length and a width of a rod of the rod array is neither a multiple nor a factor of the wavelength, and/or
wherein a distance between two adjacent rods of the rod array is neither a multiple nor a factor of the wavelength.

11. The apparatus of one of the preceding claims, wherein the light transmitted from a light source is scattered by the probe and the target of interest when the target is bound to the probe.

12. The apparatus of one of the preceding claims, wherein the apparatus is configured for scattering of light transmitted from a light source when the target of interest is bound to the probe, wherein said light is scattered in accordance with Rayleigh scattering, Mie scattering, Brillouin scattering, Raman scattering, inelastic X-ray scattering and Compton scattering.

13. A sensor comprising the apparatus of one of the preceding claims, and further comprising:
a light source that emits light;
a light receiver for receiving light; and
a detector configured to generate an electrical signal,
the magnitude of which reflects the amount of light that is received by the light receiver,
wherein the apparatus is located between the light source and the light receiver, and
wherein the apparatus is configured such that the probes are in the path of the light emitted by the light source.

14. The sensor of claim 13, wherein the light emitted from the light source comprises a wavelength of about 200nm to about 800nm.

15. A method of making an apparatus according to one of the preceding claims, comprising:
disposing the material on the substrate in a pattern configured to enhance scattering of light by the target of interest when it is bound to the probe; and
disposing a probe configured to interact with the target of interest on the material, in particular wherein the material comprises gold, silver, copper or nickel.

16. The method of claim 15, wherein the pattern comprises a rod array on the material, and/or
wherein the disposing the material further comprises annealing the material to the substrate, and/or wherein the annealing temperature is greater than 250 degrees Celsius.

17. The method of claim 15, wherein prior to the disposing of the probe, the method further comprises cleaning and pre-treating the material to facilitate the disposing of the probe on the material, in particular wherein the pre-treating includes using a compound having a thiol group or a hydroxyl group to pre-treat the material.

18. A method for using a sensor according to claim 13 for detecting a target of interest, comprising:
transmitting light from the light source through the apparatus in the absence of the target of interest, thereby generating a first electrical signal;
transmitting light from the light source through the apparatus in the presence of a sample that is suspected of containing the target of interest, thereby generating a second electrical signal; and
comparing the first electrical signal and the second electrical signal, wherein a difference in the two signals indicates that the target is present in the sample.

19. A method according to claim 18, wherein the second electrical signal is higher than the first electrical signal.
